# EUROPÄISCHE PATENTSCHRIFT

(11) **EP 2 224 978 B1**
(45) Veröffentlichungstag und Bekanntmachung des Hinweises auf die Patenterteilung: **22.02.2012**
(21) Anmeldenummer: 08866112.9
(22) Anmeldetag: 04.12.2008
(51) Int. Cl.: A61M 5/178

(54) **EINWEGINJEKTOR MIT HANDBETÄTIGBAREM KOLBEN UND EINEM ZWEIKAMMERSYSTEM**
DISPOSABLE INJECTOR WITH A MANUALLY ACTUATED PISTON AND TWO-CHAMBER SYSTEM
INSTRUMENT D'INJECTION À USAGE UNIQUE AVEC UN PISTON À ACTIONNEMENT MANUEL ET UN SYSTÈME À DEUX CHAMBRES

(30) Priorität: 01.01.2008 DE 102008003105
(43) Veröffentlichungstag der Anmeldung: 08.09.2010
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Siemund, Volker
(86) Internationale Anmeldenummer: PCT/EP2008/010251
(87) Internationale Veröffentlichungsnummer: WO 2009/083090

(56) Entgegenhaltungen:
- WO-A-03/090822
- DE-A1-102005 006 771
- US-A- 5 060 704
- US-A1- 2002 173 752

## Beschreibung

Die Erfindung betrifft einen Einweginjektor mit einem Gehäuse, einer daran angeordneten - zumindest zeitweise befüllbaren - Zylinder-Kolben-Einheit mit einem manuell bewegbaren Kolben und einem dieser Zylinder-Kolben-Einheit vorgelagerten lösbaren Behälteradapter, wobei der Behälteradapter einen - zumindest zeitweise wirkstoffbefüllbaren - mit einem öffenbaren Stopfen verschlossenen Behälter lagert.

Ein Einweginjektor gemäss dem Oberbegriff von Anspruchs 1 ist aus dem Dokument WO 03/090822 A bekannt.

Aus der WO 00/23133 ist u.a. ein derartiger Injektor bekannt. Zwischen dem lyophilisatteilbefüllten Behälter und der Zylinder-Kolben-Einheit, die hier eine vorn mittels Deckel und hinten mittels eines Kolbens verschlossene, flüssigkeitsbefüllte Kartusche ist, sitzt im Behälteradapter ein Nadelträger mit einer doppelseitigen Injektionsnadel. Durch ein vorderseitiges Einschieben des Behälters in den Behälteradapter sticht das vordere Nadelende den Verschluss des Behälters auf. Durch ein Einschrauben des Behälteradapters in den Injektor sticht das hintere Nadelende den Deckel der Kartusche auf. Zugleich wird der Nadelträger an der Kartusche dauerhaft adaptiert. Die Flüssigkeit der Kartusche wird in den Behälter gepumpt, dort löst sich das Lyophilisat. Die Lösung wird dann in die Kartusche umgepumpt. Nach dem Abnehmen des Behälteradaptes wird die Feder des Injektors - zur Schaffung der Injektionsbereitschaft - von Hand gespannt.

Der vorliegenden Erfindung liegt die Problemstellung zugrunde, einen modular aufgebauten Einweginjektor zu entwickeln, der eine Flüssigkeit und einen Wirkstoff jeweils separat steril lagert und einen Raum zur Verfügung stellt, in dem der Wirkstoff für die Applikation in der Flüssigkeit gelöst oder mit der Flüssigkeit gemischt wird. Der Injektor und der Behälteradapter sollen einen einfachen konstruktiven Aufbau haben und problemlos zu bedienen sein.

Diese Problemstellung wird mit den Merkmalen der Ansprüche 1 und 11 gelöst.

Nach Anspruch 1 ist der Kolben der Zylinder-Kolben-Einheit über eine Pumpstange separat bewegbar. Der Behälteradapter umgreift vorderseitig den Zylinder der Zylinder-Kolben-Einheit zumindest bereichsweise. Der Behälteradapter weist einen Zwischenboden auf, der an der freien Stirnseite des Zylinders zumindest bereichsweise anliegt und eine Ausnehmung für einen Doppeladapter mit mindestens einem Durchgangskanal hat. Im Behälteradapter sitzt rückseitig längsverschiebbar der Behälter, wobei er im Auslieferungszustand mit seinem Stopfen am Doppeladapter dicht anliegt und rückseitig durch eine Kappe gehalten und gesichert ist. Der Behälter ist - zum Öffnen durch Hineinstoßen des Stopfens in den Behälter - gegen den Zwischenboden verschiebbar.

Der Anspruch 11 beschreibt ein Verfahren zur Herstellung einer Lösung aus einem Lösemittel und einem Wirkstoff in und an einem Einweginjektor. Vor der Herstellung der Lösung ist das Lösemittel in einer injektorseitigen Zylinder-Kolben-Einheit gelagert, während der Wirkstoff in einem, der Zylinder-Kolben-Einheit vorgelagerten, mit einem Stopfen verschlossenen Behälter enthalten ist. Zwischen der Zylinder-Kolben-Einheit und dem Stopfen ist ein durchbohrter Doppeladapter angeordnet. Der Behälter wird - zur Herstellung einer Verbindung zwischen dem Innenraum des Zylinders der injektorseitigen Zylinder-Kolben-Einheit und dem Innenraum des Behälters - gegen den Doppeladapter unter einem Verdrängen des Stopfens verschoben. Durch das Einschieben des Kolbens strömt das Lösemittel in den Innenraum des Behälters. Dort löst sich der Wirkstoff im Lösemittel zu einer Lösung. Die Lösung wird in die Zylinder-Kolben-Einheit durch ein Zurückziehen des injektorseitigen Kolbens gepumpt.

Mit der Erfindung wird hier beispielsweise ein nadelfreier Einmalinjektor vorgestellt, dessen Zylinder-Kolben-Einheit bei gespanntem Federspeicher durch eine manuelle Pumpbewegung ihres Kolbens geleert oder befüllt werden kann. U.a. wird dazu von außen her eine Pumpstange in den im Injektor angeordneten Kolben der Zylinder-Kolben-Einheit eingekuppelt, so dass mittels der Pumpstange der Kolben bewegt werden kann. Ggf. wird die Pumpstange vor der Anwendung des Injektors ausgekuppelt und aus dem Gehäuse herausgezogen.

Der Zylinder-Kolben-Einheit ist ein Behälter vorgelagert, der in einem am Injektor befertigten Behälteradapter verschiebbar geführt ist. In der Zylinder-Kolben-Einheit wird ein Lösemittel, z.B. Wasser für Infusionszwecke, steril gelagert. In dem vorgelagerten Behälter befindet sich z.B. ein ebenfalls steril verpackter, gefriergetrockneter Arzneistoff. Unmittelbar vor der Benutzung des Einweginjektors wird das Wasser in den Behälter zum Arzneistoff gefördert. Dort bildet sich eine Lösung, eine Suspension oder eine Emulsion. Diese Flüssigkeit wird in die injektorseitige Zylinder-Kolben-Einheit umgepumpt, um dann injiziert werden zu können. Beim Umpumpen gelangen keine Verklumpungen in den Zylinderraum der ersten Zylinder-Kolben-Einheit, wodurch ein präziser Injektionsstrahl garantiert wird.

Der Behälter ist der injektorseitigen Zylinder-Kolben-Einheit hydraulisch vorgelagert. Gemäß des Ausführungsbeispiels sitzt er räumlich vor der Zylinder-Kolben-Einheit. Der Behälter kann jedoch auch seitlich am Injektor angeordnet werden. In diesem Fall würde er parallel neben der Zylinder-Kolben-Einheit sitzen.

Weitere Einzelheiten der Erfindung ergeben sich aus den Unteransprüchen und den nachfolgenden Beschreibungen schematisch dargestellter Ausführungsbeispiele.
- Figur 1:: Einweginjektor mit lösungsmittelbefüllter Zylinder-Kolben-Einheit und einem adaptierten, wirkstoffteilbefüllten Behälter;
- Figur 2:: wie Figur 1, jedoch ist das Lösungsmittel in den wirkstoffteilbefüllten Behälter gepumpt;
- Figur 3:: wie Figur 1, jedoch ist die Lösung aus dem Behälter in den Zylinder der Zylinder-Kolben-Einheit zurückgesaugt;
- Figur 4:: Einweginjektor nach dem Entfernen des Behälters, dem Entsichern und dem Betätigen (fiktiver Zustand);
- Figur 5:: wie Figur 4, jedoch nach dem Lösungsausstoß;
- Figur 6:: Einweginjektor mit lösungsmittelbefüllter Zylinder-Kolben-Einheit und einem adaptierten, wirkstoffteilbefüllten Behälter;
- Figur 7:: Behälteradapter des Einweginjektors nach Figur 6;
- Figur 7a:: Querschnitt zu Figur 7 in der Höhe des Behälterstopfens;
- Figur 8:: wie Figur 7, jedoch mit entleertem Zylinder und befülltem Behälter;
- Figur 9:: wie Figur 8, jedoch ist der Inhalt des Behälters in den Zylinder der Zylinder-Kolben-Einheit zurückgepumpt;
- Figur 10:: Einweginjektor nach dem Entfernen des Behälteradapters, dem Entsichern und dem Betätigen (fiktiver Zustand);
- Figur 11:: wie Figur 10, jedoch nach dem Lösungsausstoß;
- Figur 12:: Ausschnittvergrößerung zu Figur 8;
- Figur 13:: alternative Ausschnittvergrößerung zu Figur 8;
- Figur 14:: dimetrische Gehäuseteilansicht mit Pumpstange;
- Figur 15:: Außenansicht eines Behälteradapters mit verliersicherer Kappe und teilweise weggeschnittener Banderole;
- Figur 16:: Schnitt zu Figur 15;
- Figur 17:: wie Figur 15, jedoch mit eingeschobenem Behälter;
- Figur 18:: Schnitt zu Figur 17.

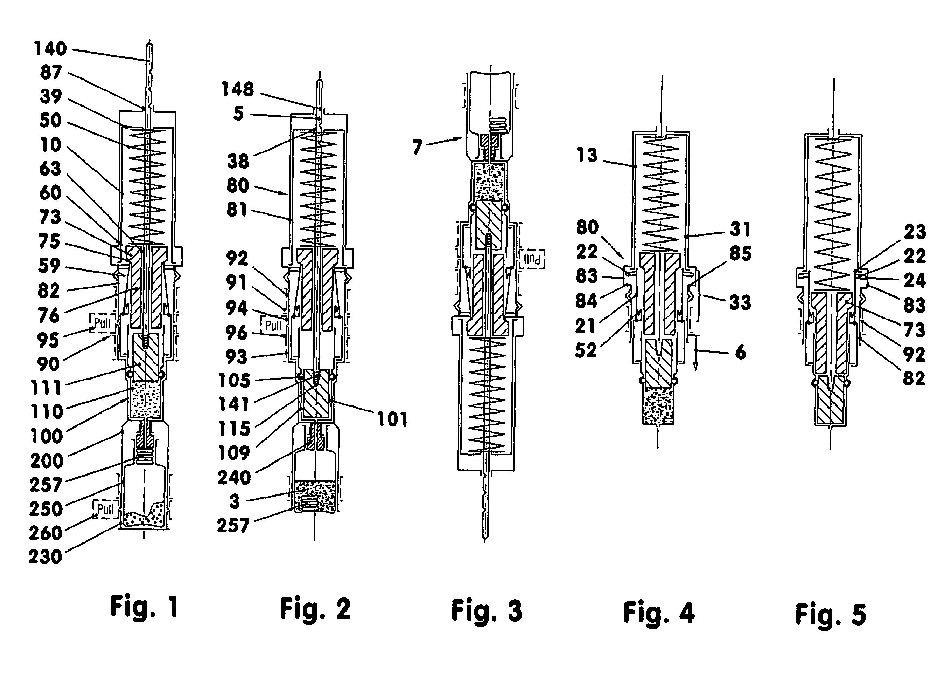

Die Figuren 1 bis 5 zeigen eine vereinfachte Prinzipskizze eines Einweginjektor-Typs mit einem dauergeladenen Federenergiespeicher mit befülltem Zylinder der Zylinder-Kolben-Einheit und adaptiertem Wirkstoffbehälter in verschiedenen Umpump- und Auslösezuständen. Der gezeigte Einweginjektor besteht aus einem Gehäuse (10), einer z.B. mit einer Injektionslösung vorbefüllten Zylinder-Kolben-Einheit (100), einem Kolbenbetätigungsstempel (60) und einer Schraubendruckfeder (50) als Federenergiespeicher. Zudem sind am Gehäuse (10) ein Auslöseelement (82) und ein Sicherungselement (90) angeordnet. Die Zylinder-Kolben-Einheit (100) ist vorn über einen Behälteradapter (200) in Kombination mit einem Doppeladapter (240) verschlossen. Die Zylinder-Kolben-Einheit (100) hat einen Kolben (111), der mittels einer separaten Pumpstange (140) im Zylinder (101) bewegt werden kann.

Im Behälteradapter (200) sitzt längsverschiebbar ein Behälter (250).

Das Gehäuse (10) ist ein topfförmiger, unten offener Hohlkörper mit obenliegendem Boden (39). Im mittleren Bereich, dem Mantelbereich (31), vgl. Figur 4, hat das Gehäuse (10) z.B. zwei einander gegenüberliegende fensterartige Durchbrüche (33). Am unteren Rand des einzelnen Durchbruchs (33) ist jeweils ein Druckstab (21) gelenkig gelagert. Der Boden (39) hat eine zentrale Bohrung (38).

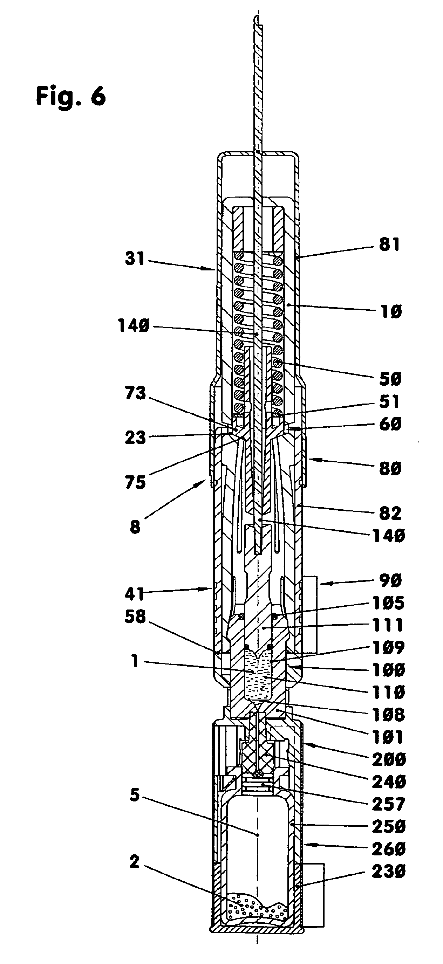

Die Druckstäbe (21) sind hier nur beispielhaft in Schwenkgelenken angeordnet und über Federelemente (52) am Gehäuse (10) abgestützt. Die Federelemente (52) drücken die Stützstäbe (21) zumindest annähernd radial nach außen gegen das Auslöseelement (82), vgl. Figur 1 bis 5. Dort liegen sie über Nocken (22) am Auslöseelement (82) an. Die Nocken (22) können dabei z.B. auch 5 bis 20 Millimeter unterhalb des jeweiligen freien, oberen Endes der Druckstäbe (21) liegen. Sind die Druckstäbe (21) am Gehäuse (10) angeformt, vgl. u.a. Figur 6, so federn sie als elastische Biegebalken (28) nach außen.

Die beiden auf Druck belasteten Druckstäbe (21) halten den Kolbenbetätigungsstempel (60) an dessen Stempelteller (73) in seiner vorgespannten Lage, vgl. Figur 1. Dazu stützen sich die Druckstäbe (21) mit ihren Abstützflächen (23) am Stempelteller (73) ab. Die Größe der jeweiligen Kontaktfläche zwischen einer Abstützfläche (23) und der entsprechenden Stelle am Stempelteller (73) liegt im Bereich von 2 bis 20 mm².

Auf der der Mittellinie (5) abgewandten Seite weist jeder Druckstab (21) an seinem Nocken (22) eine Anlagefläche (24) auf.

Im unteren Bereich des Gehäuses (10) befinden sich Halteelemente zur Befestigung der Zylinder-Kolben-Einheit (100).

Die Zylinder-Kolben-Einheit (100) besteht im Ausführungsbeispiel aus einem, mit einem Lösemittel (1), z.B. Wasser für Infusionszwecke, befüllten, transparenten Zylinder (101), in dem ein Kolben (111) in der hinteren Position sitzt. Der Zylinder (101) hat eine Zylinderinnenwandung (109), die nach hinten z.B. in einer Ringnut endet. In der Ringnut sitzt ein radial dicht am Kolben (111) anliegendes Dichtelement (105). Der Kolben (111) und das Dichtelement (105) schließen den befüllten Zylinderinnenraum (110) steril ab. Hinter der Ringnut weitet sich der Zylinder (101) so weit auf, dass ein rückwärts bewegter Kolben (111) die Wandung des aufgeweiteten Bereichs nicht kontaktieren kann.

Der Kolben (111) hat an seiner Rückseite eine z.B. zentrale, kegelstumpfmantelförmige Ausnehmung (115), in der die Pumpstange (140) mittels eines Kegelgewindes (141) eingeschraubt ist. Die Pumpstange (140) kann mit geringem Kraftaufwand vom Kolben (111) gelöst werden.

Oberhalb des Kolbens (111) ist im Gehäuse (10) der Kolbenbetätigungsstempel (60) z.B. so angeordnet, dass er den Kolben zwar nicht berührt, jedoch mit seinem unteren Ende z.B. im oberen Bereich des Zylinders (101) seitlich geführt wird. Der Kolbenbetätigungsstempel (60) hat eine z.B. zentrale Bohrung (63), die von der Pumpstange (140) mit großem Spiel durchquert wird.

Nach Figur 1 ist die untere Hälfte des Gehäuses (10) von dem hülsenartigen Auslöseelement (82) umgeben. Das Auslöseelement (82) ist auf der radialen Außenfläche (13) des Gehäuses (10) längsverschiebbar gelagert. Es endet rückwärtig mit einer scharfen Kante (85). Unterhalb der Kante (85), vgl. Figur 5, berühren nach den Figuren 1 bis 3 die Nocken (22) mit ihren außen liegenden Anlageflächen (24) sichernd die Innenwandung (59) des Auslöseelements (82).

Beispielsweise in der Nähe der Kante (85) ist am Auslöseelement (82) eine Auslösekappe (81) befestigt, die das hintere Ende des Gehäuses (10) vollständig umgibt. Dort hat sie eine zentrale Bohrung (87) zum Durchführen der Pumpstange (140). Die Auslösekappe (81) umfasst eine umlaufende Aufweitung (83), in der beim Auslösen des Injektors die Nocken (22) aufgenommen werden. Anstelle dieser Aufweitung (83) können bei einem nichtrotationssymmetrischen Auslöseelement (82) pro Druckstab (21) auch partielle Aufweitungen oder nicht abgedeckte Öffnungen vorhanden sein.

Die Aufweitung (83) ist im Bezug auf das Gehäuse (10) genau so positioniert und dimensioniert, dass sie die beim Auslösevorgang zurückweichenden, nach außen gedrängten Druckstäbe (21) mit ihren Nocken (22) aufnehmen kann. Die Innenkontur der Aufweitung (83) ist z.B. ein Kanal mit einer Rücksprungflanke (84), die hier eine zur Mittellinie (5) des Injektors normale Ebene darstellt.

Der im Gehäuse (10) angeordnete Kolbenbetätigungsstempel (60) ist in zwei Bereiche aufgeteilt. Der untere Bereich ist der Kolbenschieber (76). Sein Durchmesser ist etwas kleiner als der Innendurchmesser des hinteren Bereichs des Zylinders (101). Die untere Stirnfläche des Kolbenschiebers (76) wirkt direkt auf den Kolben (111).

Der obere Bereich des Kolbenbetätigungsstempels (60), also der Stempelteller (73), ist eine flache, zumindest bereichsweise zylindrische Scheibe, deren Außendurchmesser einige Zehntel Millimeter kleiner ist als der Innendurchmesser des Gehäuses (10) im Mantelbereich (31). Die untere Stirnseite weist eine um den Kolbenschieber (76) herum angeordnete Bundfläche (75) auf. Sie hat die Form eines Kegelstumpfmantels, dessen Spitzenwinkel ca. 100 bis 130, vorzugsweise 120 Winkelgrade beträgt. Die gedachte Spitze des Kegelstumpfmantels liegt auf der Mittellinie (5) im Bereich des Kolbenschiebers (76). Die Bundfläche (75) kann auch sphärisch gekrümmt sein.

Der Kolbenschieber (76) kann selbstverständlich auch als separates, vom Stempelteller (73) getrenntes, Bauteil ausgeführt sein. Hierzu ist er dann an der Innenwandung des Gehäuses (10) geführt.

Zwischen dem Stempelteller (73) und dem oben liegenden Boden (39) des Gehäuses (10) sitzt vorgespannt die Schraubendruckfeder (50). Die Federkraft der Schraubendruckfeder (50) wird über den Stempelteller (73) auf die Druckstäbe (21) übertragen. Aufgrund der Neigung der Bundfläche (75) werden die Druckstäbe (21) keilgetriebeartig radial nach außen gedrängt. Die Auslösehülse (82) stützt diese Radialkraft dauerhaft ab.

Unterhalb des Auslöseelements (82) befindet sich der mit einem Stopfen (257) verschlossene Behälteradapter (200), in dem der z.B. mit einem Lyophilisat teilbefüllte Behälter (250) angeordnet ist. Der Behälter (250) wird über eine Kappe (230) und eine Abreißbanderole (260) am Behälteradapter (200) gesichert. Zwischen dem Behälter (250) und dem Zylinder (101) sitzt ein durchbohrter Doppeladapter (240).

Nach einem Entfernen der Abreißbanderole (260) und der Kappe (230) wird der Behälter (250) gegen den Doppeladapter (240) verschoben, um den Stopfen (257) in den Behälter (250) zu verdrängen. Nun kann mittels der handbetätigten Pumpstange (140) der Kolben (111) in den Zylinder (101) geschoben werden, um das Lösemittel (1) in den Behälter (250) zu verdrängen. In Letzterem entsteht die Lösung (3). Nach Figur 3 wird diese in den Zylinder (101) durch ein Ziehen an der Pumpstange (140) zurückgepumpt.

Der Ansaugvorgang wird abgeschlossen, indem die ggf. in den Zylinder (101) angesaugten Gasblasen in bekannter Weise z.B. durch ein geringfügiges Zurückschieben des Kolbens (111) entfernt werden. Nun wird beispielsweise die glatte Pumpstange (140) aus der Ausnehmung (115) des Kolbens (111) herausgedreht und aus dem Gehäuse (10) herausgezogen.

Zum Entsichern des Injektors wird die Abreißbanderole (94) abgezogen, so dass die Klebeverbindung zwischen dem Behälteradapter (250) und dem Auslöseelement (82) aufgehoben ist. Der Behälteradapter (250) wird abgezogen. Der Einweg-Injektor wird auf der Injektionsstelle positioniert. Nun kann das Auslöseelement (82) in Richtung der Zylinder-Kolben-Einheit (100) verschoben werden, vgl. Figur 4. Bei diesem Vorgang gleitet das Auslöseelement (82) auf der Außenwandung (13) des Gehäuses (10) linear nach unten, also in Richtung der Injektionsstelle. Die Anlageflächen (24) der Druckstäbe (21) rutschen über die Kante (85) und springen unter der Kraft des Federelements (50) entsichernd radial nach außen in die Aufweitung (83). Der Kolbenbetätigungsstempel (60) schnellt ungehindert nach unten, vgl. Figur 5. Der Zylinder (100) wird entleert.

Anstelle einer linearen Gleitbewegung des Auslöseelements (82) auf dem Gehäuse (10) kann auch eine schraubenförmige Bewegung vorgesehen werden. In diesem Fall werden das Auslöseelement (82) und das Gehäuse (10) z.B. über einen Kulissenstein und eine Kulisse aneinander geführt. Ggf. kann das Auslösen auch durch eine reine Schwenkbewegung zwischen dem Gehäuse (10) und dem Auslöseelement (82) realisiert werden. Die Schwenkachse wäre hier die Mittellinie (5).

Die Figuren 6 bis 11 zeigen eine Ausführungsform des in den Figuren 1 bis 5 beschriebenen Prinzips. Hier ist das tragende Bauteil ein einteiliges Gehäuse (10). Es wird z.B. aus einem glasfaserverstärkten Polyamid durch Spritzgießen gefertigt. Das Gehäuse (10) hat eine weitgehend rohrförmige Gestalt und ist in zwei Funktionsbereiche aufgeteilt, das ist zum einen der obere Mantelbereich (31) und zum anderen der untere Fixierbereich (41).

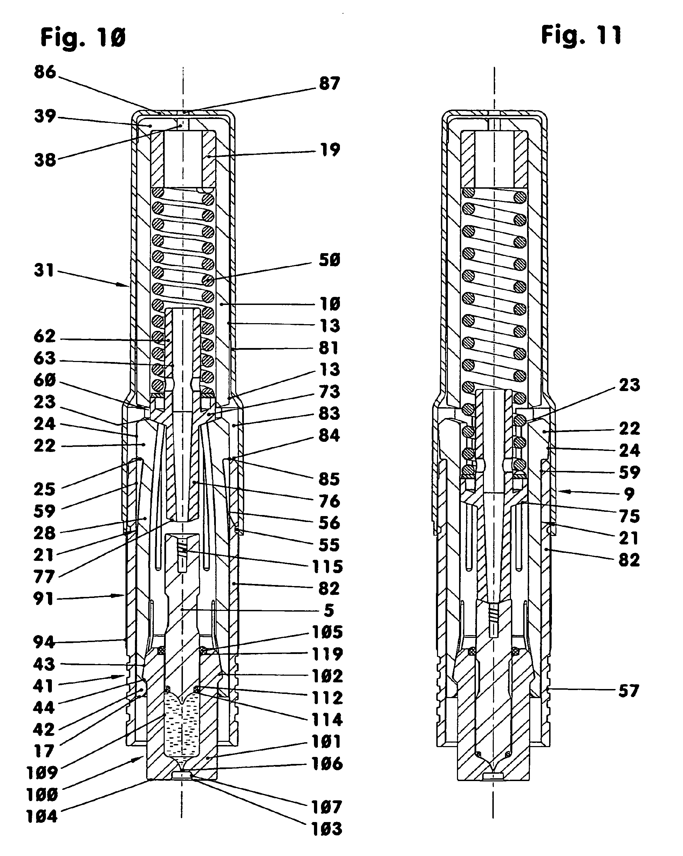

Der im wesentlichen rohrförmige Mantelbereich (31) ist oben durch einen z.B. ebenen Boden (39) mit integrierter Bohrung (38) verschlossen, vgl. Figur 10. In der unteren Hälfte des Mantelbereichs (31) befinden sich zwei einander gegenüberliegende, angeformte Druckstäbe (21). Die Anformstelle für die Druckstäbe (21) liegt knapp oberhalb des Fixierbereichs (41). Zur Ausbildung des jeweiligen Druckstabs (21) befindet sich im unteren Bereich des Mantelabschnitts (31) ein schmaler, zumindest annähernd u-förmiger Spalt, der den einzelnen Druckstab seitlich und oben umgibt. Der Druckstab (21) hat auf ca. 80% seiner Länge die Wandstärke und die Krümmung der Wandung des Gehäuses (10). Dieser Bereich hat u.a. auch die Funktion eines federelastischen Biegebalkens (28). Er hat einen sichelförmigen Querschnitt.

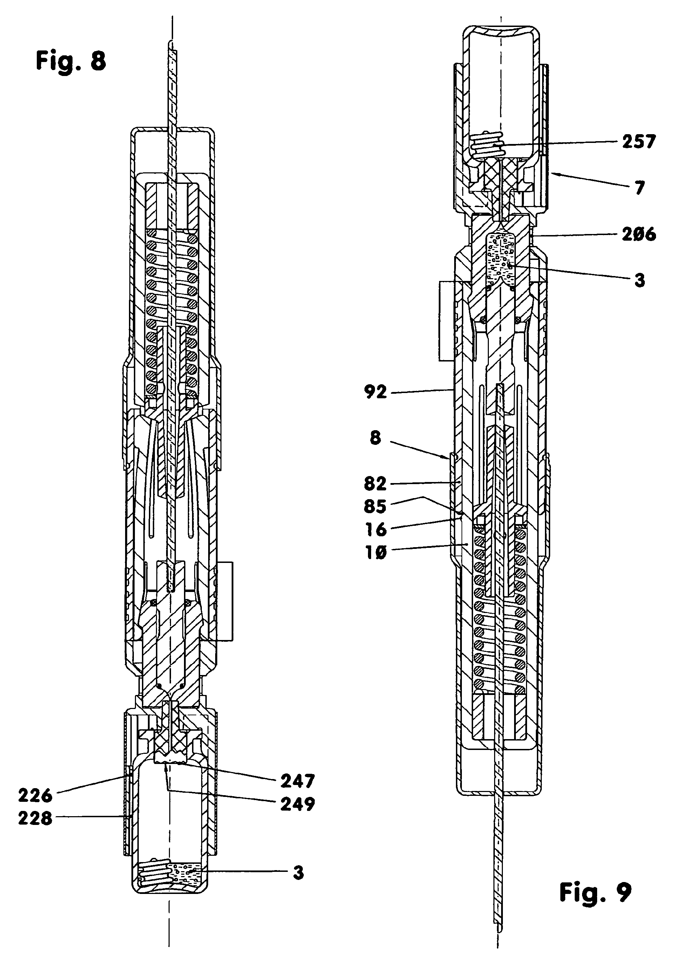

Ggf. kann ein Teil dieses Biegebalkens (28) auch mit einem rechteckigen Querschnitt ausgestattet sein, um bei der Nutzung auftretende Biegespannungen im Biegebalkenrandbereich zu reduzieren. In der Figur 8 ist der Druckstab (21) im unverformten Zustand dargestellt.

Das hier obere freie Ende des einzelnen Druckstabs (21) wird durch den radial nach außen abstehenden Nocken (22) gebildet. Letzterer hat zumindest eine Abstützfläche (23) und eine Anlagefläche (24). Nach Figur 10 liegt auf der Abstützfläche (23) der Stempelteller (73) des gespannten Einweg-Injektors über seine Bundfläche (75) auf. Die Abstützfläche (23), die hier die Funktion einer Keilfläche erfüllt, hat die Form eines Kegelstumpfmantels mit einem Spitzenwinkel von 120 Winkelgraden.

Ggf. haben die Druckstäbe (21) oder die Bundfläche (75) zumindest im Kontaktbereich eine keramische Panzerung. Ggf. ist die Bundfläche (75) durch eine z.B. aufgeklebte kegelstumpfmantelförmige Unterlegscheibe verstärkt.

Die Anlagefläche (24) der Nocken (22), vgl. Figur 11, ist Teil eines Konus, dessen maximaler Durchmesser z.B. 3 bis 4 Millimeter größer ist als der Außendurchmesser des Gehäuses (10). Die Anlagefläche (24) kontaktiert bei gespanntem Einweginjektor die Innenwandung (59) des hülsenartigen Auslöseelements (82). Ggf. hat - zur Minimierung der Flächenpressung - die Anlagefläche (24) eine Krümmung, die der Innenwandung (59) entspricht, vgl. Figur 10.

Alternativ zu den Druckstäben (21) können auch Zughaken verwendet werden, vgl. Figur 11. Diese Zughaken (21) sind im oberen Gehäusebereich angeformt. Sie umgreifen - bei gespanntem Federspeicher (50) von oben her den Stempelteller (73) des Kolbenbetätigungsstempels (60). Um beim Betätigen des Injektors dem nach außen schwenkenden Zughaken (21) genug Freiraum zu lassen, ist bei dieser Variante die Auslösekappe (81) bereichsweise kegelstumpfmantelartig ausgeführt.

Unterhalb des Mantelabschnitts (31) befindet sich der Fixierbereich (41) zur Aufnahme der einbaubaren Zylinder-Kolben-Einheit (100), vgl. Figur 10. Der Fixierbereich (41) umfasst z.B. acht parallel zur Mittellinie (5) ausgerichtete Federhaken (42). Die Federhaken (42) haben jeweils einen mindestens zweiflankigen Hintergriff zur ggf. spielfreien Aufnahme der Zylinder-Kolben-Einheit (100). Die einander gegenüber liegenden Flanken (43, 44) des Hintergriffs (42) schließen nach Figur 10 einen Winkel von z.B. 127 Winkelgraden ein. Die untere Flanke (44) hat hierbei einen Kegelwinkel von 45 Winkelgraden, dessen Kegelspitze auf der Mittellinie 5 - in Richtung Düse (106) geblickt - unterhalb der Stirnfläche (17) liegt. Die Länge und die Federrate der Federhaken (42) ist so dimensioniert, dass der Zylinder (101) ohne plastische Verformung der Federhaken (42) eingebaut werden kann.

Um das Gehäuse (10) zusammen mit dem Federelement (50) und dem Kolbenbetätigungsstempel (60) bei der Montage im Auslöseelement (82) verliersicher fixieren zu können, hat das Gehäuse (10) in einem Bereich zwischen den Nocken (22) eine linsenförmige Erhebung (16), vgl. Figur 9, über die das Gehäuse (10) an der Kante (85) des Auslöseelements (82) anliegt.

Der Zylinder (101), vgl. Figur 10, ist z.B. ein klarsichtiger, dickwandiger Topf, dessen zumindest bereichsweise zylindrische Außenwandung eine beispielsweise umlaufende Rastrippe (102) trägt, die an den Flanken (43, 44) des Hintergriffs der Federhaken (42) formsteif anliegt. Im Bereich der rückseitigen Stirnfläche des Zylinders (101) befindet sich am oberen Ende der Zylinderinnenwandung (109) ein Bund (119) zur Aufnahme eines Dichtelements (105). Ggf. kann der Bund auch eine radiale ringnutartige Eindrehung sein, die das Dichtelement (105) bereichsweise formschlüssig umgibt. Alternativ oder zusätzlich kann das Dichtelement (105) mit dem Zylinder verklebt sein. Das Dichtelement (105) ist in den Ausführungsbeispielen als O-Ring dargestellt. Es kann aber auch ein Quad-Ring, eine Lippendichtung oder ein anderes gleichwertiges Dichtelement sein.

Im Zentrum der Bohrung des Zylinders (101), dessen Zylinderboden der Kontur der vorderen Kolbenstirnseite zumindest annähernd angepasst ist, befindet sich eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie endet in einer zylindrischen oder konischen Ausnehmung (107) der bodenseitigen, äußeren Stirnfläche (103) des Zylinders (101). Die Ausnehmung (107) stellt zumindest annähernd die vorderen ein bis zwei Millimeter eines Luer-Innenkegels dar. Diese Stirnfläche (103) kann zur Erhöhung der Applikationssicherheit zusätzlich mit einem Klebering (104) versehen werden.

Der Zylinder (101) ist beispielsweise aus dem amorphen Thermoplast Cycloolefin-Copolymer (COC) hergestellt. Dieser Werkstoff ist nahezu wasserdampfundurchlässig, was eine dauerhafte Lagerung der Injektionslösung ermöglicht.

In der z.B. zylindrischen oder konischen Bohrung des Zylinders (101) sitzt der stangenlose Kolben (111). Der z.B. aus dem Teflon^{®}-Derivat Tetrafluorethylen/Hexafluorethylen-Copolymer (FEP) hergestellte Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. Die Länge des Kolbens (111) ist so gewählt, dass der eingefahrene Kolben (111), vgl. Figuren 8 oder 11, mindestens einen Millimeter über die hintere Zylinderoberkante übersteht. Der mittlere Bereich des Kolbens (111), ist tailliert ausgeführt. Der umlaufend taillierte Bereich hat eine Länge, die ca. 30% der Kolbengesamtlänge entspricht. Der taillierte Bereich hat einen Durchmesser, der 16 bis 20% kleiner ist als der maximale Zylinderinnendurchmesser im Bereich des lösungsaufnehmenden Zylinderinnenraumes (110). Der vordere Übergang, der zwischen dem taillierten Bereich und dem vorderen, also hier untenliegenden, Kolbenbereich liegt, hat z.B. einen Kegelwinkel von 35 bis 40 Winkelgraden. Der andere, hintere Übergang hat einen Kegelwinkel zwischen 35 und 90 Winkelgraden.

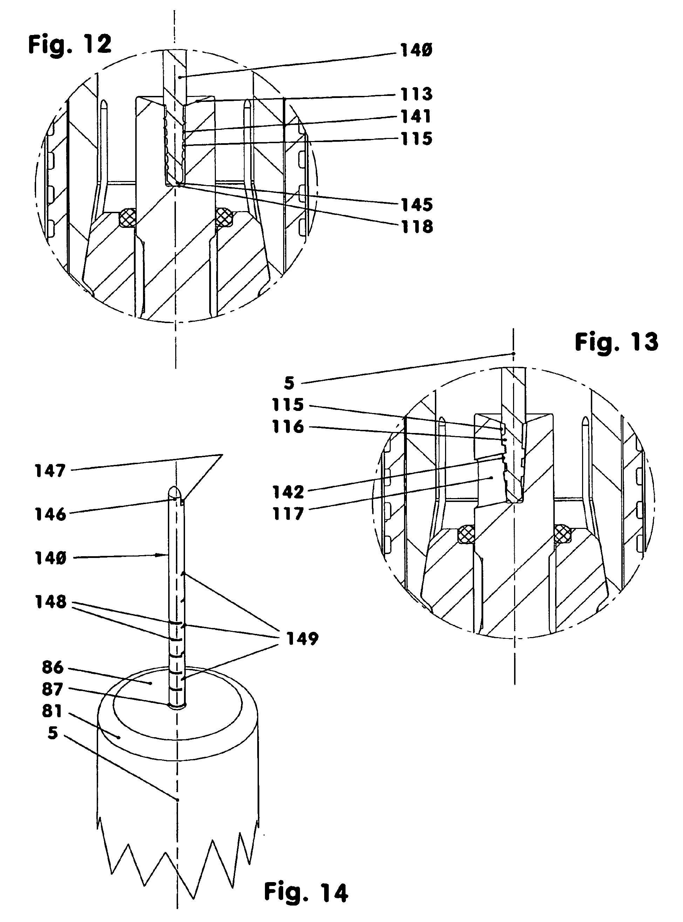

In der rückseitigen, z.B. kegelstumpfmantelförmigen Stirnfläche (113) des Kolbens (111) befindet sich eine zentrische, konische Kolbenausnehmung (115) mit dem Boden (118), vgl. Figur 12, zur Ankupplung der Pumpstange (140). Der Kegelwinkel der Kolbenausnehmung (115) beträgt z.B. ein Winkelgrad. Die Pumpstange (140) hat zum Ankuppeln am Kolben (111) an ihrem unteren Ende u.a. nach Figur 12 ein kegeliges Spitzgewinde (141). Der Kegelwinkel des Spitzgewindes (115) beträgt z.B. sechs Winkelgrade. Der Gewindegang des Spitzgewindes (141) drückt beim Eindrehen der Pumpstange (140) in die Kolbenausnehmung (115) das erforderliche Gegengewinde ein. Der Eindrehvorgang ist beendet, wenn das vordere Ende der Pumpstange (140) mit der schmalen Spitze der kegelstumpfförmigen Stirnseite (145) den Grund (118) kontaktiert.

Die Figur 13 zeigt als Kupplung zwischen der Pumpstange (140) und dem Kolben (111) ein kegeliges Trapezgewinde (142), das in eine Kolbenausnehmung (115) eingreift, in der mindestens ein Teilgang (116) oder eine Nocke angeordnet ist. Der gezeigte Teilgang (116) erstreckt sich im Querschnitt, also normal zur Mittellinie (5), über 30 bis 60 Winkelgrade. Nur über den Teilgang (116) werden die von der Pumpstange (140) auf den Kolben (111) zu übertragenden Axialkräfte weitergeleitet. Aus fertigungstechnischen Gründen befindet sich unterhalb des Teilgangs ein Schieberlangloch (117).

Beide zu Kupplungszwecken verwendeten Sondergewinde (141, 142) benötigen nur geringe Ein- und Ausschraubkräfte. Selbstverständlich können auch andere lösbare Kupplungen verwendet werden, wie z.B. ein Schlüssel/Schlüssellochsystem oder ein einfaches Rastsystem.

Die Pumpstange (140) hat über den größten Teil ihrer Länge z.B. einen gleichbleibenden Querschnitt und eine glatte Oberfläche. Ihr größter Durchmesser beträgt im Ausführungsbeispiel ca. zwei Millimeter. Sie ist z.B. aus einem glasfaserverstärkten Polyamid gefertigt. An ihrem hinteren Ende, das aus der Bohrung (87) der Auslösekappe (81) herausragt, weist sie zwei verschieden geteilte Skalen (148) und (149) auf, vgl. u.a. Figur 14. Die Skala (148) passt zu einer Zylinder-Kolben-Einheit (100), deren Zylinder (101) einen mittleren Innendurchmesser von sieben Millimetern hat, während die andere Skala (149) zu einem Zylinder (101) mit sechs Millimetern Innendurchmesser gehört. Die verschieden großen Zylinder (101) können wahlweise in den Injektor eingesteckt sein.

Beide Skalen (148, 149) haben jeweils horizontale Teilstriche. Diese Teilstriche liegen in Ebenen, die normal zur Mittellinie (5) orientiert sind. Jeder Teilstrich hat beispielsweise die Länge des halben Pumpstangenumfangs. Die Teilstriche einer Skala (148, 149) liegen alle übereinander. Sie können z.B. farbige, schwarze oder weiße Striche sein oder als Kerben in die Pumpstange eingeprägt sein. Statt der Striche können auch Punkte oder Zahlen benutzt werden.

Ggf. kann das hintere Ende der Pumpstange (140) - zur besseren Griffigkeit - mit einer Struktur versehen sein, z.B. einer Quer- oder Längsrillung, einer Riffelung oder dergleichen. Auch kann der Querschnitt - zur Massenreduktion - bereichsweise abgeflacht sein oder einen größeren Durchmesser haben, als der Bereich, der sich durch den Kolbenbetätigungsstempel (60) erstreckt.

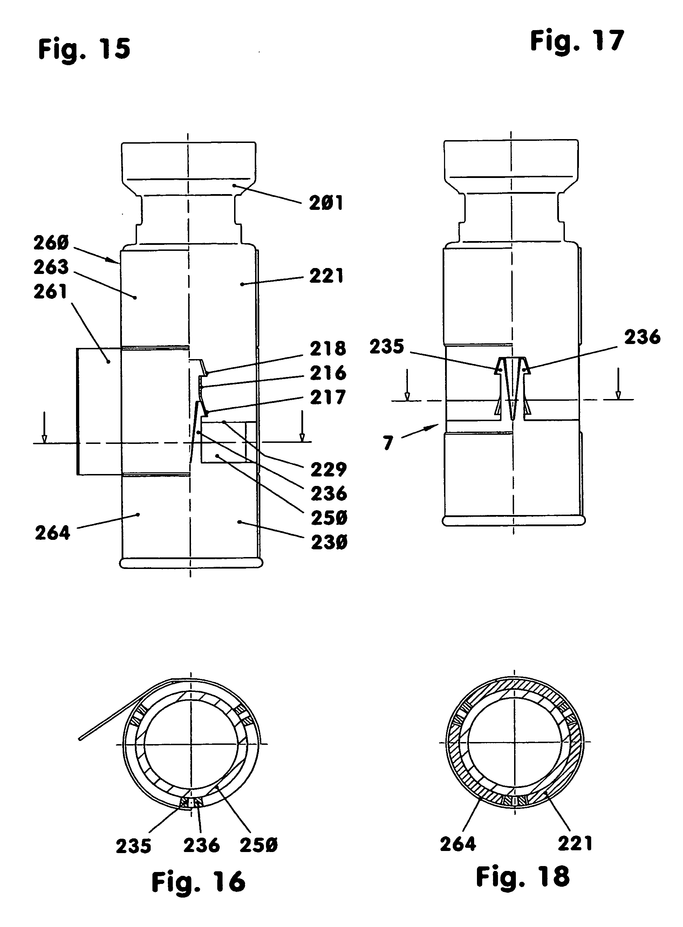

Nach Figur 16 ist die vordere, dem größeren Zylinder zugeteilte Skala (148) zur Kenntlichmachung der Zuordnung um einen Halbzylinder (146) verlängert. Die beiden unteren, direkt auf dem Niveau des Bodens (86) gelegenen Teilstriche markieren das Nennvolumen der jeweiligen Zylinder (101). Der jeweils oben liegende Teilstrich zeigt an, dass der Kolben (111) vollständig in den Zylinder (101) eingeschoben ist. Jeder andere Teilstrich der Skalen (148, 149) steht z.B. für 0,1 Milliliter.

Zwischen dem Kolben (111) und dem Boden (39) ist der Federenergiespeicher (50) bzw. die Antriebseinheit des Einweginjektors angeordnet.

Der Federenergiespeicher (50) ist eine Schraubendruckfeder, die auf dem Kolbenbetätigungsstempel (60) mit dem Stempelteller (73) angeordnet ist. Er stützt sich am oben liegenden Boden (39) des Gehäuses (10) unter Zwischenschaltung einer Distanzhülse (19) ab. Mittels des Stempeltellers (73) stützt sich der federkraftbelastete Kolbenbetätigungsstempel (60) an den Druckstäben (21) des Gehäuses (10) ab.

Der Kolbenbetätigungsstempel (60) hat oberhalb des Stempeltellers (73) einen Führungszapfen (62). Letzterer führt die Schraubendruckfeder (50) oder wird durch diese geführt. Unterhalb des Stempeltellers (73) befindet sich zentral in der Verlängerung des Führungszapfens (62) der Kolbenschieber (76), der bei einer Betätigung des Einmal-Injektors auf den Kolben (111) wirkt. Der Kolbenschieber (76) hat eine kegelmantelförmige, nach vorn gewölbte Stirnfläche (77), vgl. u.a. Figur 10. Mit dieser Stirnfläche (77) kontaktiert er die komplementär geformte Stirnfläche des Kolbens (111). Beide Kegel haben zumindest annähernd den gleichen Kegelwinkel.

Im Ausführungsbeispiel endet der Kolbenschieber (76) z.B. 2 bis 4 Millimeter oberhalb des Kolbens (111). Der Kolbenbetätigungsstempel (60) hat eine Durchgangsbohrung (63) deren obere Hälfte weitgehend zylindrisch ist, während die untere Hälfte sich nach unten hin konisch verengt. Im Bereich der unteren Stirnfläche (77) ist die Bohrung nur ein bis zwei Zehntel Millimeter größer als der Außendurchmesser der Kolbenstange.

Die Figuren 6 und 8 bis 11 stellen einen Druckstab-Injektor mit einer das Gehäuse fast vollständig umschließenden Auslöseeinheit (80) dar. Das Auslöseelement (82) ist als Teil der Auslöseeinheit (80) hier ebenfalls eine Auslösehülse. Die im Wesentlichen zylindrische, z.B. aus ABS gefertigte, Auslösehülse (82) hat als Stirnfläche die Rücksprungflanke (84) mit der innen liegenden Kante (85). An dem Auslöseelement (82) ist eine Auslösekappe (81) befestigt, die das hintere Ende des Gehäuses (10) umgibt. Die Auslösekappe (81) ist dazu über das hintere Ende des Auslöseelements (82) geschoben.

Unmittelbar oberhalb der Rücksprungflanke (84) befindet sich in der Auslösekappe (81) die Aufweitung (83). Oberhalb der Aufweitung (83) liegt die Auslösekappe (81) gleitfähig an der Außenwandung (13) des Gehäuses (10) an.

Zur Befestigung der Auslösekappe (81) am Auslöseelement (82) hat das Auslöseelement (82) beispielsweise eine Ringnut (56), in die ein Umlaufsteg oder Rastnocken (55) der Auslösekappe (81) eingreift. Ggf. ist die Auslösekappe (81) - zur Erleichterung der Montage - z.B. zweifach bereichsweise längsgeschlitzt.

Im unteren Bereich des Auslöseelements (82) befinden sich in dessen Außenwandung mehrere umlaufende Rillen (57), vgl. Figur 11, oder eine andere vergleichbare Struktur. Die Rillen (57) haben gegeneinander z.B. gleiche Abstände und erstrecken sich über 10 bis 30 Millimetern Länge des Auslöseelements (82).

Das zylindrische Auslöseelement (82) ist auf seiner gesamten Länge mit einem Klebeetikett (91) umhüllt. Das Klebeetikett (91) selbst ist z.B. ein mit einem Klebstoff bereichsweise einseitig beschichteter Papier- und/oder Folienstreifen. Der Folienstreifen umgibt z.B. einlagig einmal den Verbund aus Behälteradapter (200) und Auslöseelement (82). Er besteht als Originalitätsverschluss (90) aus drei separaten Streifen, die jeweils über eine Perforation (96) gegeneinander abtrennbar sind. Der obere Streifen ist das Hauptteil (92), der mittlere Streifen ist eine Abreißbanderole (94) mit einer zwei bis drei Zentimeter langen Abreißfahne (95) und der untere Streifen ist das Adapterteil (93). Das Hauptteil (92) und das Adapterteil (93) tragen eine Klebeschicht, mit der sie an dem Auslöseelement (82) befestigt sind.

An der unteren Stirnfläche (58) des Auslöseelements (82) liegt der am Zylinder (101) der Zylinder-Kolben-Einheit (100) zentrierte Behälteradapter (200) an. Die zumindest bereichsweise annähernd zylindrische Außenfläche des Behälteradapters (200) hat den gleichen Außendurchmesser wie die ebenfalls zylindrische Außenfläche des Auslöseelements (82) in der Nähe ihrer Stirnfläche (58).

Der Behälteradapter (200) ist ein büchsenartiges Bauteil, das einen verschiebbar gelagerten, geschlossenen Behälter (250) und einen Doppeladapter (240), z.B. einem Luer-Lock-Stopfen mit Durchgangskanal, in einem Behälterbereich (221) aufnimmt. Zugleich hat er einen hülsenförmigen Adapterbereich (201) mit dem er sich, auf dem Zylinder (101) zentriert, am Gehäuse (10) abstützt.

Der Adapterbereich (201) ist ein Becher, der zumindest das untere Viertel des Zylinders (101) eng anliegend umgibt und vor der Stirnseite (103) des Zylinders (101) in einem Zwischenboden (211) endet. Er hat eine obere, zur Stirnfläche (58) hin orientierte Stützzone (202) und eine zum Zwischenboden (211) hin ausgerichtete Fensterzone (205), die bei transparentem Adaptermaterial auch entfallen kann.

Die bündig an das Auslöseelement (82) anschließende Stützzone (202) hat z.B. drei oder fünf radial nach innen - zur Mittellinie (5) hin - ragende Anlagestege (203), die bis an die Außenwandung des Zylinders (101) heranreichen. Die Anlagestege (203) berühren die Stirnfläche (17) des Gehäuses (10).

Die taillierte Fensterzone (205) liegt an der zylindrischen Außenwandung des Zylinders (101) eng an. In ihr sind zwei einander gegenüberliegende, z.B. nahezu rechteckige Fenster (206) angeordnet. Die Fenster (206) haben eine Breite, die mindestens dem Durchmesser des Kolbens (111) entspricht. Die Fenstermitten befinden sich in der Höhe des Zylinderbodens (108), vgl. auch Figur 9. In der dort dargestellten Einweginjektorposition kann mit Hilfe der Fenster (206) im Durchlicht u.a. die Blasenfreiheit der Injektionslösung (3) kontrolliert werden.

Der z.B. ebene Zwischenboden (211) kontaktiert die Stirnfläche (103) des Zylinders (101) an dessen mit einer Folie geschützten Klebering (104) und beispielsweise über einen ringförmigen Steg (214) mit einer halbtorusförmigen Oberfläche. Der Steg (214) umrandet eine zentrale Ausnehmung (212) des Zwischenbodens (211). Die zentrale Ausnehmung (212), die durch ein Stützrohr (213) auf mindestens die dreifache Zwischenbodenwandstärke verlängert ist, hat als Innenwandung zumindest bereichsweise einen Luer-Innenkegel.

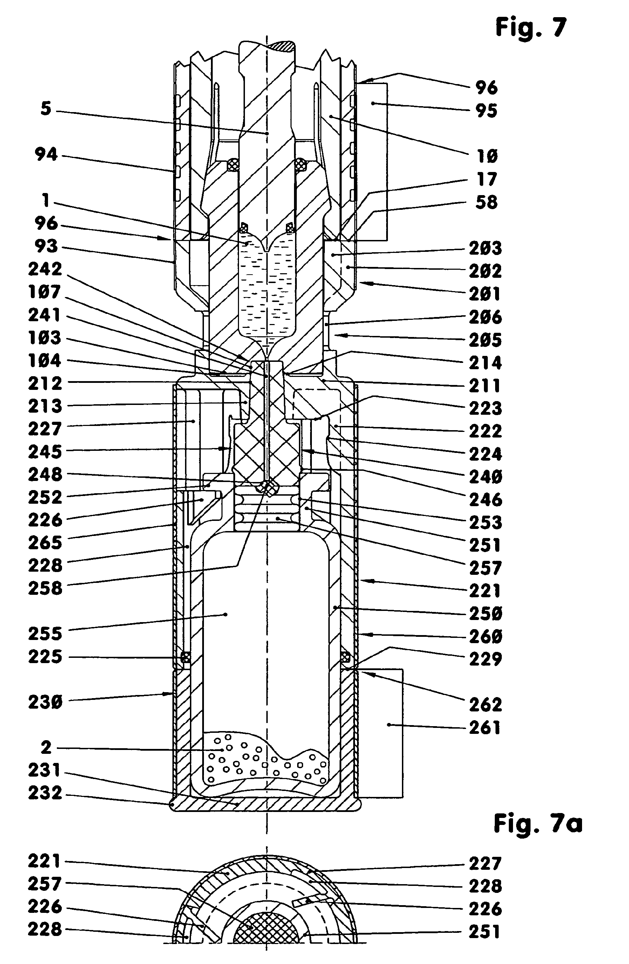

Im rohrförmigen Behälterbereich (221) wird das Stützrohr (213) von z.B. drei radial angeordneten, um je 120° geteilten Raststegen (222) umgeben. Die Raststege (222) bilden - zur späteren Fixierung des Behälters (250) - zum einen axiale Anschläge (223) und zum anderen Rasthintergriffe (224) aus, vgl. Figur 7 und 7a. Zwischen den Raststegen (222) befinden sich drei Schieberausnehmungen (227), von denen jeder einem Klapprasthaken (226) vorgelagert ist. Die zumindest annähernd dreieckigen Klapprasthaken (226), die nach den Figuren 6 und 7 den Flanschrand (252) des Behälters (250) hintergreifen, sind jeweils in einer Längsnut (228) des Behälterbereichs (221) angeordnet. Nach Figur 7a ragen sie - ausgeklappt - nach innen, wobei jeweils eine ihrer Seitenflächen in einer gedachten Ebene liegt, die den Behälterstopfen (257) tangiert. Im eingeklappten Zustand, vgl. Figur 8, schmiegen sich die Klapprasthaken (226) in die Längsnuten (228). Dort werden sie durch den Behälter (250) gehalten.

Die Schieberausnehmungen (227) werden beispielsweise mit einem gasdichten, elastischen Ventilschlauch (265) steril verdeckt.

Der Behälterbereich (221), dessen Innendurchmesser nur geringfügig größer ist als der maximale Außendurchmesser des Behälters (250), erstreckt sich nach Figur 7 bis in das untere Drittel des Behälters (250). Dort endet er z.B. in einer glatten Stirnfläche (229). Ggf. befindet sich oberhalb der Stirnfläche (229) in einer Ringnut ein elastischer Dichtring (225), der die Fuge zwischen dem Behälter (250) und der Innenwandung des Behälterbereiches (221) steril verschließt.

An die Stirnfläche (229) schließt sich eine Kappe (230) an, die den hinteren Bereich des Behälters (250) z.B. vollständig umgibt. Der Kappenquerschnitt, quer zur Mittellinie (5) gemessen, entspricht - von den Längsnuten (228) abgesehen - dem Querschnitt des Behälterbereichs (221). Die Kappe (230) hat einen Boden (231), der einige Millimeter über die radiale Kappenaußenkontur überstehen kann. Ggf. kann die Kappe (230) auch ein Gitterkörper oder nur ein Bügel sein.

Die Kappe (230) ist nach Figur 7 über eine Abreißbanderole (260) mit dem Behälterbereich verbunden. Die Abreißbanderole (260), ein Originalitätsverschluss, deckt zumindest den unteren Teil der zylindrischen Außenwandung des Behälterbereichs (221) und der Kappe (230) ab. Die Abreißbanderole (260) hat im Bereich der Stirnfläche (229) eine umlaufende Perforation (262) oder Kerbe, die als Sollbruchstelle dient. Im Bereich der Kappe (230) ist die Abreißbanderole (260) als Abreißfahne (261) ausgebildet. Wird Letztere ringsherum von der Kappe (230) entgegen der Klebehaftung - unter Auftrennung der Perforation (262) - abgewickelt, kann die Kappe (230) vom Behälter abgezogen werden. Hierbei verhindern die Klapprasthaken (226) ein Herausziehen des Behälters (250) aus dem Behälteradapter (200).

Alternativ kann zur Einsparung des Ventilschlauches (265) und des Dichtrings (225) die Abreißbanderole (260) auch die Schieberausnehmungen (227) abdecken. In diesem Fall muss die Abreißbanderole (260) die Schieberausnehmungen (227) gasdicht und steril verschließen, vgl. Figuren 6, 8 und 9.

Der Behälter (250) ist z.B. ein Glasfläschchen, bzw. ein Lyophilisatfläschchen, mit einem taillierten Hals (251) und einem Flanschrand (252). Der Flanschrand (252) steht über den Hals (251) über. Sein Außendurchmesser ist jedoch kleiner als der maximale Behälteraußendurchmesser. Der Übergang zwischen dem Hals (251) und der zylindrischen Außenwandung des Behälters (250) ist mit einem großen Radius abgerundet, der z.B. der doppelten Behälterwandstärke entspricht.

Die Öffnung (253) des Behälters (250) ist z.B. mit einem Behälterstopfen (257) aus Gummi verschlossen. Der Behälterstopfen (257) hat an seiner Oberseite einen zentralen, kegelstumpfförmigen Dichtnoppen (258).

Zwischen dem Behälter (250) und dem Zwischenboden (211) ist ein z.B. aus Gummi oder einem Elastomer gefertigter Doppeladapter (240) angeordnet. Der Doppeladapter (240), der in Längsrichtung von mindestens einem Durchgangskanal (241) durchzogen ist, sitzt nach Figur 7 zum einen mit seinem Behälterabschnitt (245) bereichsweise in der Behälteröffnung (253) und zum anderen mit seinem Luer-Lock-Abschnitt (242) in der Luer-Lock-Innenkegel-Ausnehmung (212) des Zwischenbodens (211).

Der Behälterabschnitt (245) des Doppeladapters (240) hat eine zylindrische Form, deren Durchmesser - zur Erzielung eines dichten Sitzes - geringfügig größer ist als der Innendurchmesser der Öffnung (253). Die zylindrische Außenkontur weist zusätzlich einen Umlaufsteg (246) auf. An diesem Umlaufsteg (246) liegt nach Figur 7 die Kante der Öffnung als Transportsicherung an. Die dem Behälterstopfen (257) zugewandte Stirnseite (247) des Doppeladapters (240) hat mittig eine kegelige Vertiefung (248), in die der Durchgangskanal (241) mündet und in die der Dichtnoppen (258) des Behälterstopfens (257) dichtend hineinragt.

Um die Vertiefung (248) herum weist die Stirnfläche (247) eine Struktur auf, die mit einer Hirthverzahnung vergleichbar ist, vgl. Figur 8. Die Struktur, die auch als Querrillen, Noppen und dergleichen geformt werden kann, soll verhindern, dass der Behälterstopfen (257) mit dem Doppeladapter (240) großflächig verkleben kann. Des Weiteren verhindert die Struktur auch ein unbeabsichtigtes Verschließen des Durchgangskanals (241) beim Zurückpumpen der Lösung (3), vgl. Figur 9. Ersatzweise kann auch der Stopfen (257) eine derartige Struktur tragen. Haben beide Teile (240, 257) eine Struktur, so dürfen diese nicht kompatibel sein.

Der kegelstumpfförmige Luer-Lock-Abschnitt (242) kontaktiert nahezu vollflächig mit seiner Spitze dichtend die Ausnehmung (107) des Zylinders (101). Somit bilden der Zylinderinnenraum (110) und der Durchgangskanal (241) einen steril abgeschlossenen Hohlraum.

Um den Einweginjektor benutzen zu können, muss der im Behälter (250) gelagerte Wirkstoff (2), z.B. das Lyophilisat, in der im Zylinder (101) der Zylinder-Kolben-Einheit (100) vorhandenen Flüssigkeit (1), z.B. Wasser für Injektionszwecke, bzw. Physiologische Kochsalzlösung, gelöst werden. Dazu soll die Flüssigkeit (1) in den Behälter (250) gepumpt werden.

In einem ersten Schritt wird die Abreißfahne (261) von der Kappe (230) unter einem Auftrennen der Perforation (262) entfernt und die Kappe (230) vom hinteren Teil des Behälters (250) abgezogen.

In einem zweiten Schritt wird der Behälter (250) in den Behälteradapter (200) hineingeschoben. Dabei gleitet der Behälter (250) an der Innenwandung des Behälteradapters (200) nach vorn, bis er mit dem Flanschrand (252) an den Anschlägen (223) anliegt. Gleichzeitig umgreifen die Rasthintergriffe (224) die Rückseite des Flanschrandes (252) und sichern so die vordere Position des Behälters (250). Bei der Vorwärtsbewegung hat der Behälter (250) die Klapprasthaken (226) zur Seite gedrückt, vgl. Figur 8, und die Öffnung (253) des Behälters (250) hat sich über den Umlaufsteg (246) hinweg dichtend auf den Behälterabschnitt (245) des Doppeladapters (240) geschoben. Dabei wurde der Behälterstopfen (257) nach innen aus der Öffnung (253) geschoben, so dass nun der Zylinderinnenraum (110) und der Behälterinnenraum (255) über den Durchgangskanal (241) kommunizieren.

Der beim Einschieben des Behälters (250) im Behälterbereich (221) entstehende Überdruck entweicht über den hierbei partiell abhebenden Ventilschlauch (265).

In einem dritten Schritt wird der Kolben (111) mittels der Pumpstange (140) in den Zylinder (101) geschoben und so die Flüssigkeit (1) in den nun unter leichtem Überdruck stehenden Behälterinnenraum (255) gefördert, vgl. Figur 8. Die Pumpstange (140) wird dazu in der Regel zwischen dem Zeigefinger und dem Daumen der bedienenden Hand feinfühlig gehalten.

Das Lyophilisat (2) löst sich in der Flüssigkeit (1). Der Lösevorgang kann optisch kontrolliert werden, da der aus dem Behälteradapter (200) herausragende Behälter (250) transparent ist.

In einem vierten Schritt, vgl. Figur 9, wird die neu entstandene Lösung (3) in den Zylinderinnenraum (110) zurückgepumpt. Dazu wird der Injektor so gehalten, dass die Öffnung (253) des Behälters (250) in Schwerkraftrichtung zeigt. Der Kolben (111) wird über die Pumpstange (140) in eine hintere Position gezogen. Ein blasenfreies Befüllen wird über die Fenster (206) geprüft.

In einem fünften Schritt wird zum Entsichern des Einweg-Injektors die Abreißbanderole (94) mit Hilfe der Abreißfahne (95) ringsherum vom Hauptteil (92) und vom Adapterteil (93) getrennt. Die Rillen (57) des Auslöseelements (82) werden sichtbar. Der Behälteradapter (200) wird nun nach unten vom Zylinder (101) abgezogen, vgl. Figur 10.

In einem letzten Schritt wird der Injektor auf die Injektionsstelle gesetzt und das hülsenartige Auslöseelement (82) nach unten - in Richtung der Injektionsstelle - geschoben. Die Druckstäbe (21) biegen sich elastisch nach außen in ihre eigentliche Ausgangslage. Hierbei rutschen die Nocken (22) über die Kante (85) nach außen in die Aufweitung (83). Die nun nicht mehr verformten Druckstäbe (21) geben den Kolbenbetätigungsstempel (60) frei, so dass sich der Kolben (111) unter der Wirkung des Federelements (50) ruckartig zum Entleeren des Zylinders (101) nach unten bewegt, vgl. Figur 11. Bei der Vorwärtsbewegung des Kolbens (111) vermindert sich die Kolbenreibung zwischenzeitlich, da das rückwärtige Dichtelement (105) beim Passieren des taillierten Kolbenbereiches nicht bremsend anliegt.

In den Figuren 15 bis 18 ist ein alternativer Behälteradapter (200) dargestellt. Er hat im Gegensatz zum Behälteradapter (200) nach Figur 7 eine verliersichere Kappe (230) und eine dreiteilige Banderole (260).

Der Behälterbereich (221) weist z.B. zwei oder mehr, nach den Figuren 15 bis 18 jeweils drei, Vierfachrastausnehmungen (216) auf, die sich von der Stirnfläche (229) aus in Richtung Adapterbereich (201) erstrecken. Die Vierfachrastausnehmung (216) haben je zwei einander gegenüberliegende vordere Rastkerben (217) und zwei hintere Rastkerben (218). Nach Figur 15 greifen in die vorderen Rastkerben (217) zwei Rasthaken (235, 236) ein, die einander jeweils die Rücken zuwenden. Zwischen den paarweise angeordneten Rasthaken (235, 236) befindet sich ein Freiraum, der es den Rasthaken (235, 236) ermöglicht, in die Vierfachrastausnehmung (216) eingeschoben zu werden.

Gemäß Figur 15 befindet sich der Behälter (250) in der Position, die auch in Figur 7 dargestellt ist. Die Rasthaken (235, 236) und die Freiräume zwischen den Rasthaken sind mittels der Abreißbanderole (260) überdeckt. Die Abreißbanderole (260) verhindert das Zusammenschieben der Teile (221) und (230).

In Figur 17 wird der Behälteradapter (200) im zusammengeschobenen Zustand gezeigt. Um das Zusammenschieben zu ermöglichen, wurde zuvor die Abreißfahne (261) vom Behälteradapter (200) vollständig abgewickelt. Anschließend wurde der Behälter (250) zusammen mit der Kappe (230) so verschoben, dass die Rasthaken (235, 236) in die hinteren Rastkerben (218) eingerastet sind. Der Behälter (250) hat dann die Position erreicht, die auch in Figur 8 dargestellt ist. Durch die Verrastung ist der Behälter (250) vollständig im Behälteradapter (200) eingeschlossen.

Der Behälteradapter (200) ist hier aus einem transparenten Werkstoff gefertigt, damit das Lösen des Lyophilisats beobachtet werden kann.

Ggf. werden anstatt weniger Rasthaken sehr viele verwendet, damit bei der Montage der Kappe (230) am Behälterbereich (221) ein gegenseitiges Verrasten möglich ist, ohne eine genaue Positionierung der Teile (230, 221) zueinander zu benötigen. Auch ist es denkbar, dass alle oder ein Teil der Rasthaken am Behälterbereich (221) angeordnet sind oder ist, während die entsprechenden Rastausnehmungen in die Kappe eingearbeitet sind.

Anstelle der Rasthaken (235, 236) und der Rastausnehmungen (216) kann zwischen dem Behälterbereich (221) und der Kappe (230) auch ein längsgeschlitzter Stützring angeordnet werden, der über die Abreißbanderolen gestützt wird. Hier kann dann nach dem Entfernen des Stützringes der Behälter (250) in den Behälterbereich (221) eingeschoben werden.

Bei diesen beiden Varianten sitzt der Behälter (250) ggf. mittels eines Klemmsitzes oder verklebt in der Kappe (230).

Bei Injektoren, bei denen der Kolbenbetätigungsstempel (60) im Gehäuse (10) - zumindest abschnittsweise - mit geringem Spiel geradgeführt ist und der Kolbenbetätigungsstempel (60) eine ausreichende Biegefestigkeit aufweist, kann anstatt zweier oder mehrerer Druckstäbe (21) auch nur ein einziger Druckstab (21) verwendet werden.

Bei den in den Figuren dargestellten Varianten ist die einzelne Kontaktzone zwischen dem Druckstab (21) und dem Stempelteller (73) als Flächen (23) und (75) ausgeführt, die gleitfähig einander kontaktieren. In einer besonderen Ausgestaltung kann in jeder Fläche (23) der einzelnen Druckstäbe (21) eine Walze gelagert werden, die bei einer Betätigung des Injektors an der Fläche (75) des Stempeltellers wälzgelagert, also reibungsarm, abrollt.

Mit Ausnahme der Federelemente (50, 52), einer ggf. vorhandenen Kolbenplatte und der beispielsweise vorhandenen Lagerwalzen der Stützstäbe (21) sind alle Teile der zuvor beschriebenen Einweg-Injektoren aus Kunststoffen oder kunststoff- bzw. gummiähnlichen Werkstoffen gefertigt.

### Bezugszeichenliste:

- 1: Wasser für Infusionszwecke, Lösemittel
- 2: Lyophilisat, Wirkstoff
- 3: Injektionslösung
- 5: Mittellinie des Injektors, Längsrichtung
- 6: Auslösebewegungsrichtung von (82), Abwärtsbewegung Richtungspfeil
- 7: Umpumpposition
- 8: Sperrstellung
- 9: Lösestellung, Auslösestellung

- 10: Gehäuse, einteilig
- 13: Außenfläche, zylindrisch
- 16: Erhebung, linsenförmig
- 17: Stirnfläche, vorn, unten
- 19: Distanzhülse

- 21: Druckstäbe, Stützstäbe; Zughaken
- 22: Nocken
- 23: Abstützfläche
- 24: Anlagefläche
- 25: Hintergriffsflanke
- 28: Biegebalken

- 31: Mantelbereich
- 33: Durchbrüche
- 38: Bohrung
- 39: Boden

- 41: Fixierbereich für die Zylinder-Kolben-Einheit
- 42: Federhaken
- 43: Flanke, oben
- 44: Flanke, unten

- 50: Federelement, Schraubendruckfeder, Federenergiespeicher
- 51: Unterlegscheibe, plan
- 52: Federelemente an (21)
- 55: Rastnocken
- 56: Ringnut von (82)
- 57: Rillen von (82)
- 58: Stirnfläche von (82)
- 59: Innenwandung von (82)

- 60: Kolbenbetätigungsstempel
- 62: Führungszapfen
- 63: Bohrung, Durchgangsbohrung

- 73: Stempelteller
- 75: Bundfläche, konisch
- 76: Kolbenschieber
- 77: Kolbenschieberstirnfläche, kegelmantelförmig

- 80: Auslöseeinheit
- 81: Auslösekappe
- 82: Auslöseelement
- 83: Aufweitung
- 84: Rücksprungflanke
- 85: Kante, scharfkantig
- 86: Kappenboden
- 87: Bohrung

- 90: Originalitätsverschluss, Banderole, Sicherungselement
- 91: Klebeetikett
- 92: Hauptteil von (91)
- 93: Adapterteil von (91)
- 94: Abreißbanderole
- 95: Abreißfahne
- 96: Perforationen, Sollbruchstellen

- 100: Zylinder-Kolben-Einheit
- 101: Zylinder
- 102: Rastrippe
- 103: Stirnfläche
- 104: Klebering
- 105: Dichtelement,
- 106: Bohrung, Düse
- 107: Ausnehmung in der Stirnfläche
- 108: Zylinderboden
- 109: Zylinderinnenwandung
- 110: Zylinderinnenraum

- 111: Kolben
- 112: Ringnut
- 113: Stirnseite, hinten; Konus
- 114: Dichtring, Dichtung, Dichtelement
- 115: Kolbenausnehmung, Bohrung
- 116: Teilgang, Nocke
- 117: Schieberlangloch
- 118: Ausnehmungsgrund von (115)
- 119: Bund an (101)

- 140: Pumpstange
- 141: Kegelgewinde, Spitzgewinde
- 142: Sondergewinde, Kegelgewinde
- 145: Stirnseite, kegelstumpfförmig
- 146: Halbzylinder
- 147: Absatz (für kleinen Zylinder)
- 148: Markierungen, Halbkerben für große Zylinder
- 149: Markierungen, Halbkerben für kleine Zylinder

- 200: Behälteradapter
- 201: Adapterbereich
- 202: Stützzone
- 203: Anlagestege
- 205: Fensterzone, tailliert
- 206: Fenster, beidseitig

- 211: Zwischenboden
- 212: Ausnehmung, zentral
- 213: Halterung, Stützrohr
- 214: Steg, ringförmig, halbtorusförmig
- 216: Vierfachrastausnehmung
- 217: Rastkerben, vorn
- 218: Rastkerben, hinten

- 221: Behälterbereich
- 222: Raststege
- 223: Anschläge
- 224: Rasthintergriffe
- 225: Dichtring
- 226: Klapprasthaken, Anschläge
- 227: Schieberausnehmungen
- 228: Längsnuten
- 229: Stirnfläche

- 230: Kappe
- 231: Boden
- 232: Rand
- 235: Rasthaken, links
- 236: Rasthaken, rechts

- 240: Doppeladapter, elastisch
- 241: Durchgangskanal
- 242: Luer-Lock-Abschnitt

- 245: Behälterabschnitt
- 246: Umlaufsteg
- 247: Stirnfläche
- 248: Vertiefung
- 249: Hirth-Struktur

- 250: Behälter, Ampulle
- 251: Hals
- 252: Flanschrand
- 253: Öffnung
- 255: Behälterinnenraum

- 257: Behälterstopfen, Stopfen, elastisch
- 258: Dichtnoppen, elastisch

- 260: Abreißbanderole
- 261: Abreißfahne
- 262: Perforationen, Sollbruchstellen
- 263: Randteil, hinten
- 264: Randteil, vorn
- 265: Ventilschlauch, elastisch

## Patentansprüche

1. Einweginjektor mit einem Gehäuse (10), einer daran angeordneten - zumindest zeitweise befüllbaren - Zylinder-Kolben-Einheit (100) mit einem manuell bewegbaren Kolben (111) und einem dieser Zylinder-Kolben-Einheit (100) vorgelagerten lösbaren Behälteradapter (200), wobei der Behälteradapter (200) einen - zumindest zeitweise wirkstoffbefüllbaren - mit einem öffenbaren Stopfen (257) verschlossenen Behälter (250) lagert,
**dadurch gekennzeichnet,**
- **dass** der Kolben (111) der Zylinder-Kolben-Einheit (100) über eine Pumpstange (140) direkt separat bewegbar ist,
- **dass** der Behälteradapter (200) vorderseitig den Zylinder (101) der Zylinder-Kolben-Einheit (100) zumindest bereichsweise umgreift,
- **dass** der Behälteradapter (200) einen Zwischenboden (211) aufweist, der an der freien Stirnseite (103) des Zylinders (101) zumindest bereichsweise anliegt und eine Ausnehmung (212) für einen Doppeladapter (240) mit mindestens einem Durchgangskanal (241) hat,
- **dass** im Behälteradapter (200) rückseitig der Behälter (250) längsverschiebbar sitzt, wobei er im Auslieferungszustand mit seinem Stopfen (257) am Doppeladapter (240) dicht anliegt,
- **dass** der Behälter (250) - zum Öffnen durch Hineinstoßen des Stopfens (257) in den Behälter (250) - gegen den Zwischenboden (211) verschiebbar ist.

2. Einweginjektor gemäß Anspruch 1, **dadurch gekennzeichnet, dass** der Behälteradapter (200) den Behälter (250) zwischen zwei - die jeweilige Verschiebeposition sichernde - Anschlägen (223) und (226) lagert.

3. Einweginjektor gemäß Anspruch 2, **dadurch gekennzeichnet, dass** die Anschläge (226), zur Überwindbarkeit in einer Richtung, seitlich wegklappbar sind.

4. Einweginjektor gemäß Anspruch 1, **dadurch gekennzeichnet, dass** der Zylinderinnenraum (110) des Zylinders (101) mit dem Durchgangskanal (241) des Doppeladapters (240) in Verbindung steht.

5. Einweginjektor gemäß Anspruch 1, **dadurch gekennzeichnet, dass** der Doppeladapter (240) ein elastischer Körper ist, der während der Umpumpvorgänge der Flüssigkeiten (1) und (3) den Zylinder (101) und den Behälter (250) steril - und gegenüber der Umgebung dicht - verbindet.

6. Einweginjektor gemäß Anspruch 1, **dadurch gekennzeichnet, dass** der Behälteradapter (200) im Bereich des Bodens (108) zwei einander gegenüberliegende Fenster (126) hat oder aus einem transparenten Werkstoff gefertigt ist.

7. Einweginjektor gemäß Anspruch 1, **dadurch gekennzeichnet, dass** die Pumpstange (140) den Einweginjektor auf der dem Behälteradapter (200) abgelegenen Seite durchquert und - im montierten Zustand - aus dessen hinterem Ende des Gehäuses (10) herausragt.

8. Einweginjektor gemäß Anspruch 1, **dadurch gekennzeichnet, dass** die Pumpstange (140) am Kolben (111) oder an einer Kolbenstange des Kolbens (111) lösbar ankuppelbar ist.

9. Einweginjektor gemäß Anspruch 1, **dadurch gekennzeichnet, dass** die am Kolben (111) oder an einer Kolbenstange angekuppelte Pumpstange (140) hinter dem Kolben (111) oder der Kolbenstange eine glatte Oberfläche aufweist und dort innerhalb des Injektors an allen von ihr durchdrungenen Bauteilen entweder nicht oder mit Spiel geführt ist.

10. Einweginjektor gemäß Anspruch 1, **dadurch gekennzeichnet, dass** der Behälteradapter (200) über eine Abreißbanderole (260) am Gehäuse (10) oder an einem auf dem Gehäuse (10) gelagerten Auslöseelement (82) temporär befestigt ist.

11. Verfahren zur Herstellung einer Lösung (3) aus einem Lösemittel (1) und einem Wirkstoff (2) in und an einem Einweginjektor, **dadurch gekennzeichnet,**
- **dass** vor der Herstellung der Lösung (3) das Lösemittel (1) in einer injektorseitigen Zylinder-Kolben-Einheit (100) gelagert ist, während der Wirkstoff (2) in einem, der Zylinder-Kolben-Einheit (100) vorgelagerten, mit einem Stopfen (257) verschlossenen Behälter (250) enthalten ist,
- **dass** zwischen der Zylinder-Kolben-Einheit (100) und dem Stopfen (257) ein durchbohrter Doppeladapter (240) angeordnet ist,
- **dass** der Behälter (250) - zur Herstellung einer Verbindung zwischen dem Innenraum (110) des Zylinders (101) und dem Innenraum (255) des Behälters (250) - gegen den Doppeladapter (240) unter einem Verdrängen des Stopfens (257) verschoben wird,
- **dass** das Lösemittel (1) in den Innenraum (255) des Behälters (250) - durch das direkte Einschieben des Kolbens (111) - überströmt und sich dort der Wirkstoff (2) im Lösemittel (1) zu einer Lösung (3) löst und
- **dass** die Lösung (3) in die Zylinder-Kolben-Einheit (10) durch ein direktes Zurückziehen des Kolbens (111) gepumpt wird.

12. Verfahren gemäß Anspruch 11, **dadurch gekennzeichnet, dass** die Lösung (3) eine Suspension oder eine Emulsion ist.

## Claims

1. Disposable injector with a housing (10), with an at least intermittently fillable cylinder/piston unit (100) which is arranged thereon and has a manually movable piston (111), and with a detachable container adapter (200) mounted upstream of this cylinder/piston unit (100), wherein the container adapter (200) supports a container (250) which can be filled at least intermittently with active substance and which is closed by an openable stopper (257), **characterized in that**
- the piston (111) of the cylinder/piston unit (100) is directly movable separately via a pump rod (140),
- the container adapter (200) engages at the front at least partially around the cylinder (101) of the cylinder/piston unit (100),
- the container adapter (200) has an intermediate floor (211) which bears at least partially on the free end face (103) of the cylinder (101) and which has a recess (212) for a dual adapter (240) having at least one through-channel (241),
- the container (250) sits longitudinally displaceably in the rear of the container adapter (200) and, in the delivery state, bears tightly with its stopper (257) on the dual adapter (240),
- the container (250) is opened by displacing it towards the intermediate floor (211) and pushing the stopper (257) into the container (250).

2. Disposable injector according to Claim 1, **characterized in that** the container adapter (200) supports the container (250) between two abutments (223) and (226) which secure the respective displacement position.

3. Disposable injector according to Claim 2, **characterized in that** the abutments (226) can be folded laterally aside in order to permit movement past them in one direction.

4. Disposable injector according to Claim 1, **characterized in that** the cylinder interior (110) of the cylinder (101) communicates with the through-channel (241) of the dual adapter (240).

5. Disposable injector according to Claim 1, **characterized in that** the dual adapter (240) is an elastic body which, during the transfer pumping of the liquids (1) and (3), connects the cylinder (101) and the container (250) in a sterile manner and such that they are sealed off from the environment.

6. Disposable injector according to Claim 1, **characterized in that** the container adapter (200), in the area of the floor (108), has two windows (126) lying opposite each other or is made of a transparent material.

7. Disposable injector according to Claim 1, **characterized in that** the pump rod (140) extends through the disposable injector at the end remote from the container adapter (200) and, in the assembled state, protrudes from the rear end of the housing (10).

8. Disposable injector according to Claim 1, **characterized in that** the pump rod (140) can be coupled detachably to the piston (111) or to a piston rod of the piston (111).

9. Disposable injector according to Claim 1, **characterized in that** the pump rod (140) coupled to the piston (111) or to a piston rod has a smooth surface behind the piston (111) or the piston rod and there, inside the injector, is guided with play on all the structural parts through which it passes.

10. Disposable injector according to Claim 1, **characterized in that** the container adapter (200) is temporarily secured on the housing (10), or on a trigger element (82) mounted on the housing (10), via a tear-off banderole (260).

11. Method for preparing a solution (3) from a solvent (1) and an active substance (2) in and on a disposable injector, **characterized in that**
- before the solution (3) is prepared, the solvent (1) is stored in an injector-side cylinder/piston unit (100), while the active substance (2) is contained in a container (250) which is mounted upstream of the cylinder/ piston unit (100) and which is closed by a stopper (257),
- a dual adapter (240) with a bore extending through it is arranged between the cylinder/piston unit (100) and the stopper (257),
- to establish a connection between the interior (110) of the cylinder (101) and the interior (255) of the container (250), the container (250) is displaced towards the dual adapter (240) and forces the stopper (257) out,
- the solvent (1) flows into the interior (255) of the container (250), by the direct pushing in of the piston (111), and the active substance (2) dissolves there in the solvent (1) to form a solution (3), and
- the solution (3) is pumped into the cylinder/ piston unit (100) by direct pulling back of the piston (111).

12. Method according to Claim 11, **characterized in that** the solution (3) is a suspension or an emulsion.

## Revendications

1. Injecteur à usage unique comprenant un boîtier (10), une unité cylindre-piston (100) - pouvant être au moins temporairement remplie - disposée sur celui-ci, avec un piston déplaçable manuellement (111) et un adaptateur de récipient (200) détachable monté avant cette unité cylindre-piston (100), l'adaptateur de récipient (200) supportant un récipient (250) fermé avec un bouchon (257) pouvant être ouvert - pouvant être au moins temporairement rempli d'ingrédient actif, **caractérisé en ce que**
- le piston (111) de l'unité cylindre-piston (100) peut être déplacé séparément directement par le biais d'une tige de pompe (140),
- l'adaptateur de récipient (200) vient en prise du côté avant au moins en partie autour du cylindre (101) de l'unité cylindre-piston (100),
- l'adaptateur de récipient (200) présente un fond intermédiaire (211) qui s'applique au moins en partie contre le côté frontal libre (103) du cylindre (101) et a un évidement (212) pour un double adaptateur (240) avec au moins un canal de passage (241),
- le récipient (250) repose du côté arrière de manière déplaçable en longueur dans l'adaptateur de récipient (200), en s'appliquant dans l'état de livraison avec son bouchon (257) hermétiquement contre le double adaptateur (240),
- et **en ce que** le récipient (250) - pour l'ouverture par enfoncement du bouchon (257) à l'intérieur du récipient (250) - peut être déplacé contre le fond intermédiaire (211).

2. Injecteur à usage unique selon la revendication 1, **caractérisé en ce que** l'adaptateur de récipient (200) supporte le récipient (250) entre deux butées (223) et (226) fixant la position de déplacement respective.

3. Injecteur à usage unique selon la revendication 2, **caractérisé en ce que** les butées (226), afin de pouvoir être surmontées dans une direction, peuvent être rabattues latéralement.

4. Injecteur à usage unique selon la revendication 1, **caractérisé en ce que** l'espace interne de cylindre (110) du cylindre (101) est en liaison avec le canal de passage (241) du double adaptateur (240).

5. Injecteur à usage unique selon la revendication 1, **caractérisé en ce que** le double adaptateur (240) est un corps élastique, qui, pendant les opérations de pompage des liquides (1) et (3), relie le cylindre (101) et le récipient (250) de manière stérile - et hermétique par rapport à l'environnement.

6. Injecteur à usage unique selon la revendication 1, **caractérisé en ce que** l'adaptateur de récipient (200) présente dans la région du fond (108) deux fenêtres opposées l'une à l'autre (126) ou est fabriqué à partir d'un matériau transparent.

7. Injecteur à usage unique selon la revendication 1, **caractérisé en ce que** la tige de pompe (140) traverse l'injecteur à usage unique du côté opposé à l'adaptateur de récipient (200) et - dans l'état monté - fait saillie hors de l'extrémité arrière du boîtier (10).

8. Injecteur à usage unique selon la revendication 1, **caractérisé en ce que** la tige de pompe (140) peut être accouplée de manière détachable au piston (111) ou à une tige de piston du piston (111).

9. Injecteur à usage unique selon la revendication 1, **caractérisé en ce que** la tige de pompe (140) accouplée au piston (111) ou à une tige de piston présente derrière le piston (111) ou la tige de piston une surface lisse et n'y est pas guidée ou est guidée avec jeu à l'intérieur de l'injecteur sur tous les composants qu'elle traverse.

10. Injecteur à usage unique selon la revendication 1, **caractérisé en ce que** l'adaptateur de récipient (200) est fixé temporairement par le biais d'une bandelette de déchirure (260) sur le boîtier (10) ou sur un élément décollable (82) monté sur le boîtier (10).

11. Procédé de préparation d'une solution (3) constituée d'un solvant (1) et d'un ingrédient actif (2) dans et sur un injecteur à usage unique, **caractérisé en ce que**
- le solvant (1), avant la préparation de la solution (3), est stocké dans une unité cylindre-piston (100) du côté de l'injecteur, tandis que l'ingrédient actif (2) est contenu dans un récipient (250) fermé avec un bouchon (257), monté avant l'unité cylindre-piston (100),
- entre l'unité cylindre-piston (100) et le bouchon (257) est disposé un double adaptateur (240) traversé d'un alésage,
- le récipient (250) - pour la réalisation d'une connexion entre l'espace interne (110) du cylindre (101) et l'espace interne (255) du récipient (250) - est poussé contre le double adaptateur (240) en repoussant le bouchon (257),
- le solvant (1) déborde dans l'espace interne (255) du récipient (250) - par l'enfoncement direct du piston (111) - et l'ingrédient actif (2) s'y dissout dans le solvant (1) pour former une solution (3) et
- **en ce que** la solution (3) est pompée dans l'unité cylindre-piston (10) par un retrait direct du piston (111).

12. Procédé selon la revendication 11, **caractérisé en ce que** la solution (3) est une suspension ou une émulsion.
